# EUROPEAN PATENT APPLICATION

(11) **EP 2 996 059 A1**
(43) Date of publication of application: **16.03.2016**
(21) Application number: 14460056.6
(22) Date of filing: 12.09.2014
(51) Int. Cl.: G06F 19/24, G06F 19/18

(54) **Device for automatic testing of selection in genes with increased detection accuracy**

(71) Applicant: Politechnika Slaska, 44-100 Gliwice (PL)
(72) Inventor: Cyran, Krzysztof, 44-295 Lyski (PL)
(74) Representative: Ziolkowska, Urszula

(57) **Abstract**

A device for automatic testing of selection in genes with increased detection accuracy, **is characterised by** comprising digital input for putting a file with haplotypes of chosen gene, which is connected with integrated module of battery of neutrality tests **MBNT,** and said module of battery of neutrality tests **MBNT** comprises cache memory **CM** and is connected with system bus **SB,** which is additionally connected with interaction module **IM** comprising keyboard **KEY** and display **LCD,** as well as with non-erasable memory **NM,** primary classification module **PCM,** and final classification module **FCM,** moreover, said primary classification module **PCM** comprises unit of rule-based method choice **URMC** and programming logic array **PLA,** while said final classification module **FCM** comprises unit of connectionist method choice **UCMC** and optoelectronic unit **OU,** and in addition said primary classification module **PCM** is connected directly with said final classification module **FCM,** as well as with work mode switch **WMS,** which commutes outputs of said module of battery of neutrality tests **MBNT** to drive either inputs of said primary classification module **PCM,** or inputs of said final classification module **FCM.**

In another embodiment, a device for automatic testing of selection in genes with increased detection accuracy, **is characterised by** comprising digital input for putting a file with haplotypes of chosen gene, which is connected with integrated module of battery of neutrality tests **MBNT,** and said module of battery of neutrality tests **MBNT** comprises cache memory **CM** and is connected with system bus **SB,** which is additionally connected with interaction module **IM** comprising keyboard **KEY** and display **LCD,** as well as with non-erasable memory **NM** and with extended classification module **ECM** which comprises unit of method choice **UMC,** programming logic array **PLA,** and optoelectronic unit **OU,** and in addition said extended classification module **ECM** is connected with said module of battery of neutrality tests **MBNT,** classification mode flip-flop **CMF,** and primary result flip-flop **PRF,** whereas outputs of said extended classification module **ECM** are connected with z inputs of said classification mode flip-flop **CMF** and said primary result flip-flop **PRF,** and outputs of these flip-flops are backward connected with input of said extended classification module **ECM,** while output of said module of battery of neutrality tests **MBTN** is connected with input of said extended classification module **ECM.**

## Description

The invention is a device for automatic testing of selection in genes with increased detection accuracy.

In the state-of-the-art, the technology of "high-throughput" sequencing is known, which in cancer treatment allows to obtain sequences from individual tumour cells more and more economically. By using said device for automatic testing of selection in genes, medical personnel will receive additional picture of selection in development of tumour from which the samples have been taken. Currently existing solutions, such as catalogues of genes responsible for cancer development or catalogues of "driver" mutations supply only partial answer, because they are unable to support individual approach. The need to solve the problem, which will make possible recognition of selection operating at genes level as well as "driver" mutations in particular tumour, will contribute to better understanding of processes responsible for its growth. The genome of cancer cells is shaped by processes of mutations and selection. The first occurs typically in unstable fragile sites of chromosomes, and the second constitutes major evolutionary force in recessive cancer genes responsible for cancer growth.

It has been noted surprisingly that it is crucial to asses presence of selection operating at particular genes level in clonal cancer cells in order to discern "passenger" mutations, i.e. mutations casually not related with cancer process and evenly distributed across genome or accumulated in fragile regions, from "driver" mutations, in regions implicated in cancer development.

Te goal of the said invention is to make the procedure of testing natural selection operating at genes level, more efficient and simpler, as well as to assure high level of detection of such selection. The said invention may be a part of other testing devices.

The said device according to the invention is characterised in that it comprises digital input for reading a file with hyplotypes of the chosen gene, which is connected with integrated module of battery of neutrality tests, and the said module of battery of neutrality tests comprises cache memory and is connected with system bus, which is also connected with interaction module comprising keyboard and display, as well as with non-erasable memory, primary classification module, and final classification module, whereas said primary classification module comprises unit of rule-based method choice and programming logic array, and said final classification module comprises unit of connectionist method choice and optoelectronic unit, and in addition said primary classification unit is directly connected with said final classification module, as well as with work mode switch, which commutes outputs of said module of battery of neutrality tests to drive either inputs of said primary classification module or inputs of said final classification module.

In other embodiment, the device according to invention is characterised in that it comprises digital input for reading a file with hyplotypes of the chosen gene, which is connected with integrated module of battery of neutrality tests, and the said module of battery of neutrality tests comprises cache memory and is connected with system bus, which is also connected with interaction module comprising keyboard and display, as well as with non-erasable memory and extended classification module, which comprises unit of method choice, programming logic array, and optoelectronic unit, and in addition said extended classification module is connected with said module of battery of neutrality tests, classification mode flip-flop, and primary result flip-flop, whereas outputs of said extended classification module are connected with z inputs of said classification mode flip-flop and said primary result flip-flop, and outputs of these flip-flops are backward connected with input of said extended classification module, while output of said module of battery of neutrality tests is connected with input of said extended classification module.

The advantage of the solution according to invention is possibility of achieving increased accuracy of detection of selection operating at genes level, and therefore the outcomes of testing will be characterised by increased credibility. Due to constantly decreasing costs of "high-throughput" sequencing methods, the device according to invention will make possible application of individual approach by enabling better description of the sample under consideration taken out of the tumour in the context of present in this sample "driver" and "passenger" mutations. Another advantage of the solution according to invention is simplicity of its use, limited to: supplying digital input of said device with a file with haplotypes, which have been detected in a process of sequencing single tumour cells; supplying haplotype frequencies; choosing, by using keyboard and display, the battery of neutrality tests out of tests stored in non-erasable memory of the device; choosing method of primary and final classification; and reading the result on the device display.

The device according to invention is to be primarily used in professional medical laboratories for implementation of the procedure of testing of selection in chosen genes analysed based on material taken form the cancer tumour. After sequencing single cells from the tumour under investigation, medical laboratories by using said invention will be able to verify if given DNA region is influenced by "driver" mutation, i.e. is casually related with the tumour growth because it is under selection pressure, or, being selectively neutral, it is a fragment with increased rate of "passenger" mutations. Performing the test using device according to invention, will make possible attribute the results to individual donor of the genetic material. The device according to invention is a multiple use device, it allows for presenting results for many genes under consideration. As an example, for a gene, which undergone the natural selection process, said device displays: "SELECTION PRESENT" or other message having identical meaning.

The invention has been presented in examples of embodiments in a figure, in which fig. 1 shows the external view of said device for testing natural selection at genes level, and fig. 2 shows block schematic of said device for automatic testing of natural selection at genes level, whereas fig. 3 presents an alternative block schematic of the same device.

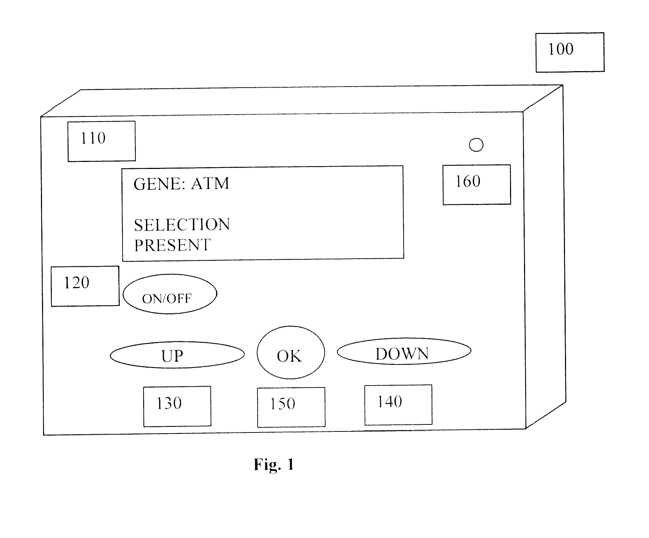

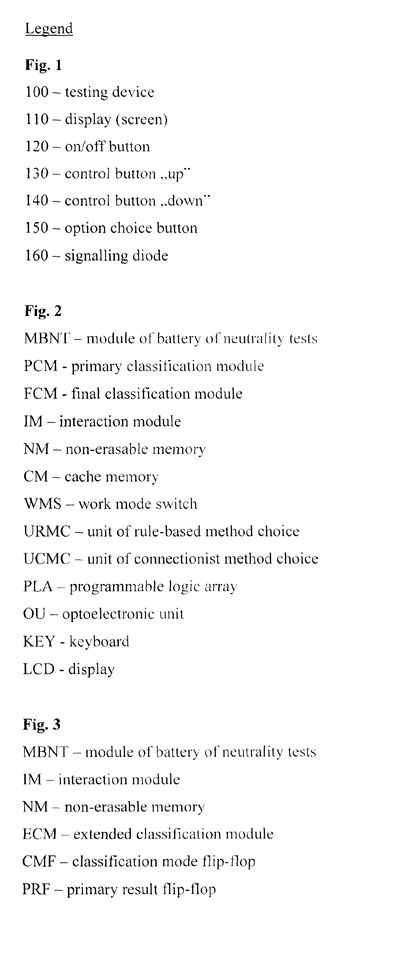

The cover of the device according to invention, presented in fig. 1 consists of display 110, on/off button 120, control button "up" 130, control button "down" 140, option choice button 150 and signalling diode 160. Said testing device 100 may be used by supplying with haplotypes with their frequencies for given gene. User activates said device 100 by pressig said on/off button 120. On a said display 110 there is a screen of options, which allows the user to choose neutrality tests which will be used, method of primary classification, method of final classification, gene code, and information concerning operation of said testing device. As an example, when there are constraints limiting type of input data for particular neutrality tests, the user may choose category of neutrality tests appropriate for the data at disposal and the gene code. The user controls option of the screen by control button "up" 130, and control button "down" 140. Control button "up" 130 and control button "down" 140 make possible to scroll options on the screen, while option choice button 150 makes possible to confirm the option indicated on the screen.

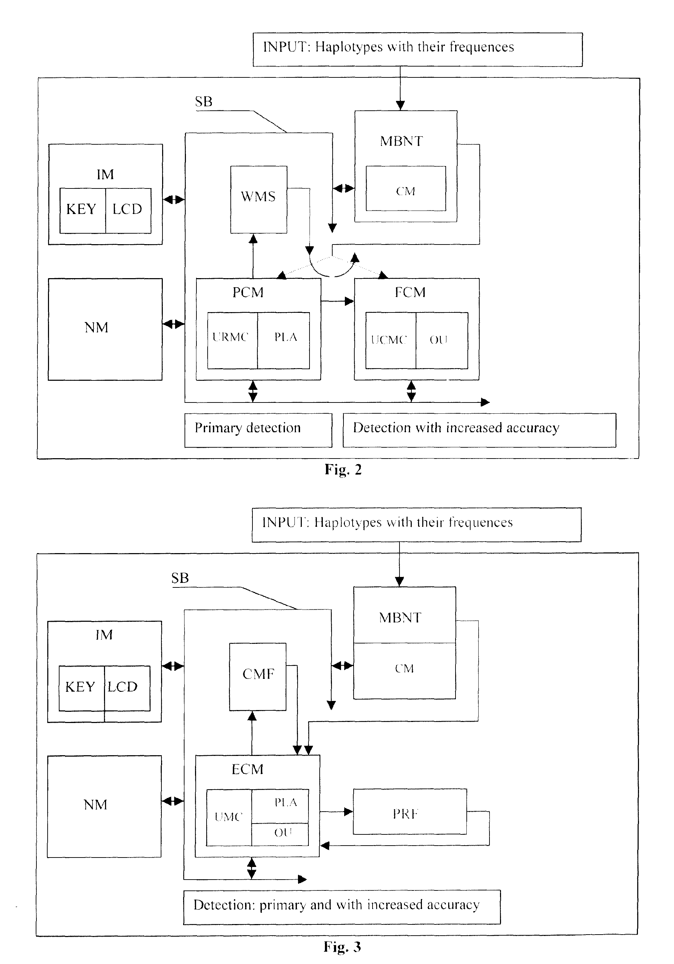

Device according to invention presented in fig. 2 comprises digital input for putting a file with haplotypes of chosen gene, detected in a process of sequencing of single cells taken from the tumour, together with their frequencies, and said input is connected with integrated module of battery of neutrality tests **MBNT,** and said module of battery of neutrality tests **MBNT** implements on-chip a battery of neutrality tests. Said module of battery of neutrality tests **MBNT** comprises cache memory **CM,** which serves for storing input information about haplotypes present in a sample under consideration as well as about their frequencies, and said module is connected to system bus **SB,** which is connected additionally with interaction module **IM,** which serves for implementing interactive operation: choice of particular tests out of neutrality tests known in state-of-the-art, choice of method of primary classification implemented by primary classification module, and choice of method of final classification implemented by final classification module. In addition, said interaction module **IM** comprises keyboard **KEY** and display **LCD**, connected by said system bus **SB** with non-erasable memory **NM**, and with primary classification module **PCM** and final classification module **FCM**, whereas said primary classification module **PCM** comprises unit of rule-based method choice **URMC** and programming logic array **PLA**, and said final classification module **FCM** comprises unit of connectionist method choice **UCMC** and optoelectronic unit **OU,** and in addition said primary classification unit **PCM** is connected directly with said final classification module **FCM**, as well as with work mode switch **WMS**, which toggles outputs of said module of battery of neutrality tests **MBNT** to drive either inputs of said primary classification module **PCM**, or inputs of said final classification module **FCM.** Due to known in the state-of-the-art non-uniqueness of single neutrality tests, in the said device a three-step processing is applied, in the following modules: **MBNT**, **PCM**, and **FCM** interconnected in such a way that said primary classification module **PCM** is controlled exclusively by output signals of said module of battery of neutrality tests **MBNT**, whereas said final classification module **FCM** is controlled both, by output signals of said module of neutrality tests **MBNT** and by output signal of said primary classification module **PCM.** In order to avoid activation of said classification module **FCM** in a moment when output signal from said primary classification module **PCM** has not been yet generated, in the device according to invention the work mode switch **WMS** is used, which activates said primary classification module **PCM,** and therefore, output signals of said module **MBNT** are transferred exclusively to the inputs of said module **PCM,** and then, in a moment when said module **PCM** has already generated result, the information about it is backward transferred to said work mode switch **WMS,** which in this moment activates said module **FCM** driving its inputs by output signals from said module **MBNT.** The number of output signals of said module of battery of neutrality tests **MBNT** is equal to the number of neutrality tests implemented in said device. Procedures of on-chip implementation of these tests are stored in non-erasable memory **NM** of aid device. Single output signal of said module of battery of neutrality tests **MBNT** is a signal which can take a value 0. 1. or 2 depending on whether a test associated with this signal generates result of no statistical significance, statistical significance, or strong statistical significance. Due to activation of said primary classification module **PCM**, by means of said work mode switch **WMS**, said primary classification module, **PCM** implements on-chip classification of signals obtained from said module of battery of neutrality tests **MBNT** using one of rule-based classification methods implemented in a structure of said programming logic array, which is the first unit of said primary classification module **PCM**. The second unit of said primary classification module **PCM** is a unit of rule-based method choice **URMC**, controlled by said interaction module **IM**, connected with said primary classification module **PCM** by said system bus **SB** of said device. After generating result of primary classification, said primary classification module **PCM** by controlling work mode switch **WMS**, initialises said final classification module **FCM** comprising optoelectronic unit **OU** and unit of connectionist method choice **UCMC**. Te purpose of said optoelectronic unit **OU** is to implement fast classification using optical elements for computation dot product required in connectionist classification. Furthermore, said unit of connectionist method choice **UCMC**, analogously to said unit of rule-based method choice **URMC**, is controlled by said interaction unit **IM** connected with said final classification module **FCM** by system bus **SB**, and it serves to choose one out of stored in said non-erasable memory **NM** on-chip procedures of connectionist classification supported by said optoelectronic unit **OU**, in order to achieve time effective operation of said device. Said device implements on-chip fusion of information generated by said primary classification module **PCM** with information from said module of battery of neutrality tests **MBNT**, in-such a way that inputs of said final classification module **FCM** are driven by output signals from said module of battery of neutrality tests **MBNT** supplemented by signal from said primary classification module **PCM**, in particular by binary signal set to 0 for selection primarily undetected by said primary classification module **PCM** or 1 for selection primarily detected by said primary classification module **PCM**. Therefore, for chosen by a user gene, said final classification module **FCM** generates on-chip result with increased accuracy of detection, and furthermore, due to usage of such unit as said programming logic array **PLA** and said optoelectronic unit **OU**, this process is implemented very time efficiently. Then, said device signals the result by setting the status of the gene to SELECTION PRESENT or SELECTION NOT PRESENT and by presenting this result on a display of said interaction module **IM**.

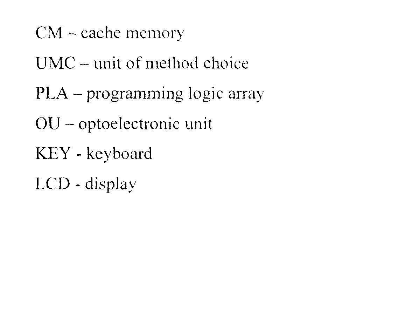

In another embodiment, presented in fig. 3 device according to invention comprises digital input for putting a file with haplotypes of chosen gene, which is connected with integrated module of battery of neutrality tests **MBNT**, and said module of battery of neutrality tests **MBNT** comprises cache memory **CM** and is connected to system bus **SB**, which is connected additionally with interaction module **IM**, which comprises keyboard **KEY** and display **LCD**, non-erasable memory **NM**, and extended classification module **ECM**, which comprises unit of method choice **DMC**, programming logic array **PLA**, and optoelectronic unit **OU**, and in addition said extended classification module **ECM** is connected with said module of battery of neutrality tests **MBNT**, clssification mode flip-flop **CMF**, and primary result flip-flop **PRF**, whereas, outputs of said module **ECM** are connected with inputs of said classification mode flip-flop **CMF** and said primary result flip-flop **PRF**, while outputs of these flip-flops are backword connected with inputs of said module **ECM**, and output of said module **MBTN** is connected with input of said module **ECM**. Said unit of method choice **UMC** is a composition of units: unit of rule-based method choice and unit of connectionist method choice, which are present in the basic version of said device in primary classification module and final classification module, respectively. In the case when said classification mode flip-flop **CMF** has got 1 on its output, then said extended classification module **ECM** implements method of primary classification storing the result in said primary result flip-flop **PRF**, which is connected with one of inputs of said extended classification module **ECM**, and changing value of said classification mode flip-flop **CMF** to 0, what informs said extended classification module **ECM**, that it should implement method of final classification using information from additional input about the primary classification result, stored in said primary result flip-flop **PRF**, and moreover it should signal result of final classification stage on said display **LCD** of said interaction module **IM**.

Device according to invention uses battery of neutrality tests and two-step classification method, where in primary processing, the rule-based classification is used, in particular classification based on expert systems, and then in final processing the connectionist classification method is used, in particular classification based on pre-trained artificial neural networks used in such a way that processing leading to final classification is implemented based on results from battery of neutrality tests and result of primary classification, hence detection with increased accuracy is achieved and the result of final processing is signalled as detection or not detection of selection in a tested gene. Moreover, in said device, there are used haplotypes with their frequencies determined after collecting results of sequencing single cells from the analysed tumour, after what data is transmitted to said device used by testing personnel.

## Claims

1. A device for automatic testing of selection in genes with increased detection accuracy, **characterised in that** it comprises digital input for putting a file with haplotypes of chosen gene, which is connected with integrated module of battery of neutrality tests **MBNT**, and said module of battery of neutrality tests **MBNT** comprises cache memory **CM** and is connected with system bus **SB**, which is additionally connected with interaction module **IM** comprising keyboard **KEY** and display **LCD**, as well as with non-erasable memory **NM**, primary classification module **PCM**, and final classification module **FCM**, moreover, said primary classification module **PCM** comprises unit of rule-based method choice **URMC** and programming logic array **PLA**, while said final classification module **FCM** comprises unit of connectionist method choice **UCMC** and optoelectronic unit **OU**, and in addition said primary classification module **PCM** is connected directly with said final classification module **FCM**, as well as with work mode switch **WMS**, which commutes outputs of said module of battery of neutrality tests **MBNT** to drive either inputs of said primary classification module **PCM**, or inputs of said final classification module **FCM.**

2. A device for automatic testing of selection in genes with increased detection accuracy, **characterised in that** it comprises digital input for putting a file with haplotypes of chosen gene, which is connected with integrated module of battery of neutrality tests **MBNT**, and said module of battery of neutrality tests **MBNT** comprises cache memory **CM** and is connected with system bus **SB**, which is additionally connected with interaction module **IM** comprising keyboard **KEY** and display **LCD**, as well as with non-erasable memory **NM** and with extended classification module **ECM** which comprises unit of method choice **UMC**, programming logic array **PLA**, and optoelectronic unit **OU**, and in addition said extended classification module **ECM** is connected with said module of battery of neutrality tests **MBNT**, classification mode flip-flop **CMF**, and primary result flip-flop **PRF**, whereas outputs of said extended classification module **ECM** are connected with z inputs of said classification mode flip-flop **CMF** and said primary result flip-flop **PRF**, and outputs of these flip-flops are backward connected with input of said extended classification module **ECM**, while output of said module of battery of neutrality tests **MBTN** is connected with input of said extended classification module **ECM**.
